# EUROPEAN PATENT APPLICATION

(11) **EP 4 495 262 A1**
(43) Date of publication of application: **22.01.2025**
(21) Application number: 23306260.3
(22) Date of filing: 21.07.2023
(51) Int. Cl.: C12Q 1/6883

(54) **HOST DNA AS A BIOMARKER OF VAGINAL HEALTH**

(71) Applicant: Luxia Scientific, 77590 Bois le Roi (FR)
(72) Inventor: The designation of the inventor has not yet been filed
(74) Representative: Regimbeau

(57) **Abstract**

The present invention relates to an *in vitro* method for diagnosing Bacterial Vaginosis (BV) in a subject comprising: a) obtaining a biological sample from said subject, b) analysing the relative abundance of the host DNA in said sample and c) diagnosing that said subject suffers from bacterial vaginosis if said relative abundance is lower than a reference value.

The present invention also relates to an *in vitro* method for diagnosing the severity of the vaginal dysbiosis in a subject, comprising the steps of: a) obtaining a biological sample from said subject, b) analysing the relative abundance of the host DNA in said sample and c) diagnosing that said subject has a severe vaginal dysbiosis if said relative abundance is lower than another reference value.

## Description

### Technical field of the invention

The invention relates to a new method for diagnosing patients with bacterial vaginosis, and/or for diagnosing the overall health status of the vagina.

### Background of the invention

Bacterial vaginosis (BV) is a common condition that occurs when there is an overgrowth of certain types of bacteria in the vagina, which can lead to an imbalance in the normal bacterial flora. BV is not considered a sexually transmitted infection (STI), although it can be associated with sexual activity. In a healthy vagina, there is a delicate balance between "good" bacteria (such as *Lactobacillus)* and "bad" bacteria. When this balance is disrupted and the bad bacteria outnumber the good bacteria, BV can occur. The exact cause of BV is not fully understood, but there are several factors that can increase the risk of developing it. These include:
- Sexual activity (although it can also occur in women who are not sexually active)
- Douching or using vaginal sprays
- Using an intrauterine device (IUD) for contraception
- Using certain antibiotics
- Having a weakened immune system

Symptoms of BV may include a fishy odor, itching, burning, or a thin grayish-white discharge from the vagina. However, some women with BV may have no symptoms at all.

BV is typically diagnosed through a pelvic exam and a laboratory test of vaginal secretions. The Nugent score is a method used to diagnose bacterial vaginosis (BV) based on the evaluation of Gram-stained vaginal smears. The method involves examining a sample of vaginal discharge under a microscope and counting the number of different types of bacteria present. The scoring system assigns a score based on the presence and quantity of three different bacterial morphotypes, with a score of 7-10 indicating BV, a score of 4-6 indicating intermediate flora, and a score of 0-3 indicating normal flora.

While the Nugent score can be a useful tool in diagnosing BV, it has some limitations:
- Subjectivity: The Nugent score is based on visual interpretation of bacterial morphology, which can be subjective and dependent on the experience and training of the person performing the analysis.
- False negatives: In some cases, women with BV may have a low Nugent score or even a normal score, which can lead to a false negative diagnosis.
- False positives: Conversely, women who do not have BV may have a high Nugent score, which can lead to a false positive diagnosis.
- Lack of information about vaginal inflammation: The Nugent score does not provide information about vaginal inflammation, which is an important factor in diagnosing and treating BV.
- Limited information about the vaginal microbiome: The Nugent score only provides information about a small subset of bacterial species present in the vaginal microbiome, and may not accurately reflect the overall composition of the microbiome.

The rate of false positives in BV diagnosis based on Nugent scoring can vary depending on a number of factors, including the skill of the laboratory technician performing the scoring and the quality of the Gram-stained vaginal smear being evaluated. However, studies have reported false positive rates ranging from 5% to 15%, which means that a small percentage of women may be diagnosed with BV when they do not actually have the condition based on Nugent scoring alone. To overcome some of these limitations, healthcare providers may use additional tests and assessments, such as pH testing, vaginal fluid analysis, and clinical symptoms, to diagnose BV and guide treatment decisions. Treatment may include antibiotics, such as Metronidazole, Secnidazole or Clindamycin, which can be taken orally or applied topically. The local or oral use of pre, pro or post-biotics have also been shown to be beneficial for vaginal microbiome health.

It's important to note that BV is not a serious health condition and is generally easily treatable. However, if left untreated, it can lead to other complications such as increased risk of sexually transmitted infections, pelvic inflammatory disease, and preterm labor in pregnant women (Ravel *et al.* 2021). The estimated annual global economic burden of treating symptomatic BV is US $4.8 billion. The US economic burden of BV is nearly tripled when including costs of BV-associated preterm births and human immunodeficiency virus cases. The prevalence of BV varies widely depending on the population studied and the diagnostic criteria used, but it is estimated that the prevalence in the general population ranges from 23% to 29% across regions (Peebles *et al.* (2019)).

The tight control of BV, through accurate surveillance and treatment adjustment, is thus a key in the management of such patients, because of the high recurring rate of BV (50% at 6 month and 80% at 12 months). Yet, none of the current diagnostic methods is satisfactory, for the above-mentioned reasons.

Patients and healthcare providers are moreover actively looking for self-collection tools enabling evaluation of disease activity and monitoring of patients care.

More precisely, there remains a need to identify a biomarker of BV that would allow diagnosing BV in a patient in a way that is simple and accurate and that supports self-collection. This is precisely the subject of the present invention.

Also, there is a need for identifying a biomarker which could help in distinguishing within intermediate stage female patients those who are at increased risk of evolving into clinical BV, from those who are not. Indeed, this information could help clinicians in diagnosing the stage of BV, predicting the occurrence of said changes, in order to choose from the different treatment options (intensive or conventional, use of pre or probiotics...). This need is also fulfilled by the present invention.

The increase of host DNA in stool samples of patients suffering from diseases known to induce an inflammatory state of the gut epithelial mucosa has been observed in the prior art.

For example, host DNA proportions were found to be negatively correlated with intestinal microbiota diversity in a prospective cohort of 599 hospitalized patients, by analysing a single rectal swab obtained within 7 days of their admission in the hospital. *Enterococcus* and *Escherichia* were enriched in patients excreting high quantities of human DNA, while *Ruminococcus* and *Odoribacter* were depleted. The quantification of human DNA in faeces was proposed to serve as a biomarker of risk of Clostridium difficile (Vincent et al, 2014).

In addition, stool samples from colorectal cancer patients have also been reported to contain increased concentrations of human DNA (Klaassen et al 2003).

Finally, stool samples from Crohn's Disease patients have been shown to contain increased concentrations of human DNA (WO2016/120475). It was later also reported by Jiang et al. 2020.

However, the amount of host DNA in vaginal smears obtained from BV suffering patients has never been assessed so far. The present inventors observed, for the first time, that vaginal smears from BV patients contain decreased concentrations of human DNA. This decrease is very surprising given the inflammatory nature of BV and the prior art reports mentioned above.

It is even more surprising given that previous studies have described bacterial vaginosis (BV) as associated with increased cell-shedding from the cervicovaginal epithelium. Cell-shedding in excess of cell-proliferation is thought to decrease epithelial barrier function and to increase susceptibility to infection. A previous study evaluated the number of shed cells in mid-vaginal smears from women with a diagnosis of symptomatic BV (sBV, n = 17), asymptomatic BV (aBV, n = 71), or no BV (n = 104) by Amsel criteria. The sBV smears contained significantly more shed cells (median 158/100X field) than no BV smears (median 91/100X field), p = 7.2e-9. (O'Hanlon *et al.* 2020)

The present inventors analysed, by a quantitative metagenomic analysis, the human DNA abundance in a number of vaginal swabs from women that have been collected from healthy controls and BV patients. Moreover, the host DNA abundance was assessed in vaginal swabs obtained from patients suffering from BV diagnosed by a Nugent score greater than 7 vs. by a Nugent score lower than 3.

In so doing, the present inventors observed that the presence and quantity of human DNA into vaginal smears are markers of bacterial vaginosis, and that healthy patients can be discriminated using this very same biomarker.

To sum up, it is herein demonstrated for the first time that the relative abundance of host DNA in the vaginal smears of women can be used as an efficient biomarker of vaginal health, of vaginal dysbiosis or disease severity. Importantly, the assessment of this biomarker can be easily performed remotely, and extemporaneously, after self-collection of the samples by the women.

### Figure Legends

**Figure 1** discloses the boxplots of the percent human DNA found in vaginal smears from BV and healthy patients.
**Figure 2** discloses the negative relationship between vaginal pH and percent human DNA. In particular a steady decrease in percent human DNA is observed when the pH is greater than 5. A high pH is indicative of BV severity.

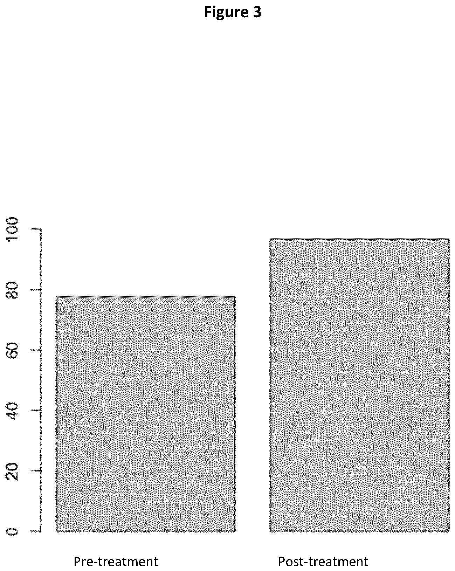

**Figure 3** discloses the rebalancing of the vaginal eco-system following a one week intervention with a daily ovule of a post-biotics (Bactigyn, CCD Laboratories) as assessed by the increase of percentage of human DNA.

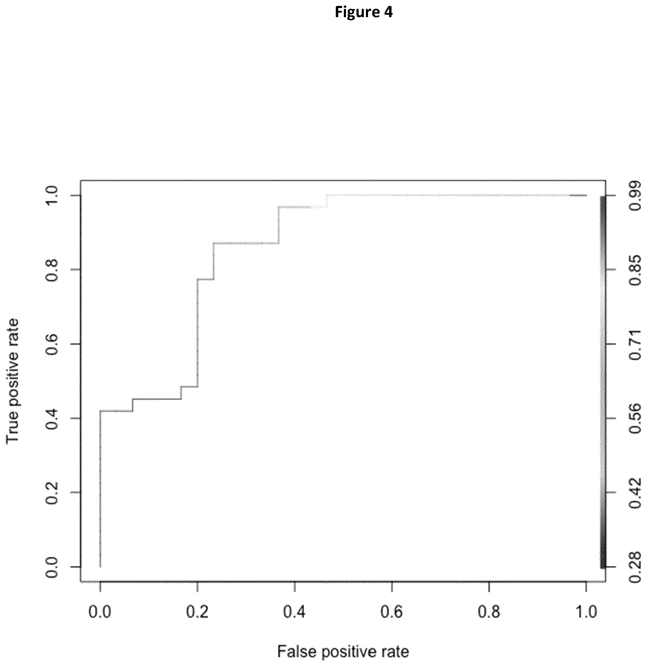

**Figure 4** discloses ROC curve using the percent of human DNA versus BV diagnosis by Nugent Score.

### Detailed description of the invention

### Methods to diagnose BV

In a first aspect, the present invention relates to an *in vitro* method for diagnosing Bacterial Vaginosis (BV) in a subject, said method comprising:
a) Obtaining a sample of vaginal microbiota from said subject,
b) Determining the relative abundance of host DNA in said sample,
   and
c) Diagnosing that said subject suffers from Bacterial Vaginosis, if said relative abundance is lower than a reference value.

This method is advantageous over the prior art diagnosis method as it supports self-collection of the samples and transfer of same to remote analysis laboratories that can perform the determination steps and diagnostics extemporaneously and remotely. Moreover, the method is economically acceptable and present a high positive predictive value.

As used herein, the term **"host DNA"** refers to the DNA of the host of the vaginal microbiota, as opposed to microbial or viral DNA. If the tested subject is a human patient, then the term "host DNA" refers specifically to "human DNA".

DNA can be extracted from said biological sample of interest for example by using the ZymoBIOMICS^{™} DNA Miniprep Kit which is designed for purifying DNA from a wide array of sample inputs. The ZymoBIOMICS^{™} innovative lysis system eliminates bias associated with unequal lysis efficiencies of different organisms (e.g. Gramnegative/positive bacteria including endospores2, fungi, protozoans, algae, etc.), making it ideal for microbial community profiling. (ZymoResearch) Other protocols can nevertheless be used and are well-known. Of note, the microbial DNA and the host DNA do not need to be physically separated for subsequent analysis.

Mammal DNA can be distinguished from microbial DNA by any conventional mean, such as detection of CpG methylation or of the bacterial 16S ribosomal DNA. It is also possible to use qPCR targeting the ALU (STR) repeat regions in human DNA, or the Beta-globulin, Beta-actin, and hTERT genes (Klaassen CHW et al, 2003; Shewale JG et al, Journal of Forensic Science, 2007, vol.52(2)).

Quantification of the host and microbial DNA can be performed by any well-known method. The most commonly used methods known in the art for the quantification of DNA strands in a sample include Northern blotting and *in situ* hybridization (Parker & Barnes, Methods in Molecular Biology 106:247-283 (1999)) and PCR-based methods, such as quantitative polymerase chain reaction (qPCR) (Heid et al., Genome Research 6:986-994 (1996)). Alternatively, antibodies may be employed that can recognize sequence-specific duplexes, including DNA duplexes or DNA-protein duplexes. Representative methods for sequencing-based analysis include chain-termination methods, shotgun sequencing methods, *de novo* sequencing, next generation sequencing methods (including Massively Parallel Signature Sequencing (MPSS), Polony sequencing, 454 pyrosequencing, Illumina (Solexa) sequencing, SOLiD sequencing, Ion semiconductor sequencing, DNA nanoball sequencing, Helioscope single molecule sequencing, Single molecule real time (SMRT) sequencing, RNAP sequencing, Nanopore DNA sequencing, Sequencing by hybridization and Microfluidic Sanger sequencing).

As shown in the examples below, it is also possible to measure host DNA from a pool of DNA by i) sequencing the DNA present in the vaginal smear using high throughput sequencing technologies and ii) by aligning the reads obtained by means of these sequencing technologies to the human genome. In this case, "relative abundance of host DNA" can be calculated by counting the number of reads mapped to a single reference sequence from the human genome (H) and the remaining number of reads generated (B), and normalizing the number of reads H by the total amount of reads (H+B).

As meant herein, the term **"host DNA abundance"** refers to the **relative amount** of host DNA as compared with the total amount of DNA present in said sample (including in particular bacterial and fungal DNA). In the present application, it will therefore preferably be referred to as **"relative abundance"** (or "relative amount") of host DNA.

Preferably, the host DNA abundance is measured by qPCR with human specific primers.

The method of the invention can be applied to any female **subject,** either human or animal. Yet, in a preferred embodiment, it is applied to a human patient, in particular to a woman who is suspected of suffering from BV, or is in need of a confirmation of having BV. More precisely, the method of the invention is useful for monitoring human women showing clinical symptoms such as increased vaginal discharge, fishy smell or itching.

In a preferred embodiment, the **sample** used in step a) of the method invention is a vaginal swab. The collection of this sample can be performed by a practician but it is preferably done by the woman herself. This sampling is completely harmless. Preferably, said vaginal sample is placed and stored in appropriate buffers that do not denature or affect the DNA contained in same (in this aim, one can use, e.g., the DNA/RNA Shield by Zymo Research, RNA later^{®} RNA stabilization Reagent (Ambion), or Smart eNat (Copan)). More preferably, the samples are stored at -80°C until DNA extraction and subsequent analysis.

As used herein, the term **"reference value"** refers to a specific value or dataset that can be used to identify samples that are known to be abundant in host DNA. This reference value is obtained for example from the historical abundance data obtained for healthy women or female subject. It can be a single cut-off value, such as a median or mean. It can be a single number, equally applicable to every sample. In a preferred embodiment, this reference value is a predetermined value. Typically, this predetermined value is of about 80%, preferably of about 75%, more preferably of about 70%.

In principle, vaginal samples of healthy women have very high levels of host DNA. Therefore, the reduction of host DNA in the vaginal swab of a subject is a hint that said subject may suffer from a severe vaginal dysbiosis. The present invention also encompasses all methods aimed at diagnosing BV in a subject, involving the detection of the reduction of host DNA in a vaginal swab. In other words, any diagnostic method involving the use of host DNA as biomarker of BV is encompassed within the present invention.

In a preferred embodiment, it can be concluded that the tested subject is suffering from BV if the relative abundance of host DNA, as defined above, is lower than 80%, preferably lower than 75%, more preferably lower than 70%.

In other terms, in the method of the invention, the amount of host DNA is **compared** with a reference value. Said comparison can be done by those skilled in the art using statistical methods, in particular a ROC curve can be used to determine an optimal cut-off for sensitivity and specificity.

In a particular embodiment, the method of the invention comprises the following steps:
a) Obtaining a vaginal swab from a woman,
b) Determining the relative abundance of human DNA in said sample, by any conventional means disclosed above,
   and
c) Diagnosing that said patient suffers from bacterial vaginosis, if said relative abundance is lower than about 80%.

The present invention therefore encompasses all methods aimed at diagnosing the presence of BV in a subject, involving the detection of the presence of host DNA in vaginal microbiota samples. In other words, any diagnostic method of BV involving the use of host DNA as biomarker is encompassed within the present invention.

### Methods to monitor the states of Vaginal dysbiosis

The results obtained by the inventors allowed to identify a biomarker (host DNA) allowing to distinguish between patients suffering from BV or not. The same biomarker can also be used to detect the severity of vaginal dysbiosis or BV states.

The host DNA in the sample of vaginal microbiota correlates to the vaginal pH which is a well-known marker of vaginal dysbiosis severity.

In another aspect, the present invention targets a method aiming at diagnosing these severity states in a subject suffering from vaginal dysbiosis.

More precisely, the present invention relates to an *in vitro* method for diagnosing the severity of the vaginal dysbiosis in a subject, said method comprising the steps of:
a) Obtaining a sample of vaginal microbiota from said subject,
b) Determining the relative abundance of host DNA in said sample,
   and
c) Determining the vaginal dysbiosis severity that said subject suffers from, by said relative abundance.

All the embodiments disclosed above for the diagnostic method of the invention also applied to the present method that is aimed at monitoring the severity of the BV.

In a preferred embodiment, the relative abundance of host DNA is determined as disclosed above.

It can be concluded that the patient suffers from very severe bacterial vaginosis, if said relative abundance is lower than about 60%, and extremely severe if said relative abundance is lower than about 50%.

The present inventors have found that the method of the invention is highly sensitive and specific when the relative abundance of host DNA is determined and compared, directly or indirectly, to a reference value.

The methods of the invention can include (or exclude) the steps consisting of obtaining the microbiota sample and extracting the nucleic acid molecule from said sample, as defined above.

### Methods to design a treatment

In another embodiment, the diagnostic methods of the invention can be used for **adapting a treatment** for a woman or a female subject suffering from bacterial vaginosis.

In this embodiment, the methods of the invention therefore comprise the additional step of designing a treatment for the diagnosed subject, said treatment being adapted to the particular state of BV which has been diagnosed (by the method of the invention).

More preferably, in this embodiment, the invention encompasses a **method for treating** a subject suffering from bacterial vaginosis, said method comprising the following steps:
i) diagnosing the activity of BV in a subject according to the above-mentioned method, and
ii) treating said subject with an appropriate treatment, said appropriate treatment being chosen in those classically attributed by the practitioner once said state of BV is diagnosed.

For example, if a BV patient is diagnosed in a severe state, an adapted treatment can be a pharmacological treatment such as metronidazole, clindamycin, secnidazole, dequalinium chloride combined with a pre, pro or post-biotic.

Alternatively, if a BV patient is diagnosed in a mild state, an adapted treatment will be for example lifestyle interventions, and only pre, pro or post-biotics.

Moreover, it is possible to use the methods of the invention for **testing the efficiency of a treatment** in a subject suffering from BV, or to evaluate the response of a patient to a treatment.

In this embodiment, the method of the invention comprises the following steps:
i) diagnosing the activity of BV before and after the administration of a treatment, according to the above-mentioned method,
   and
ii) concluding that the treatment is efficient in said subject if the state before the administration of the treatment was severe but becomes mild upon administration of the treatment.

If the BV patient is diagnosed to be "severe" before the administration of the treatment and becomes "mild" or "intermediate" upon administration of the treatment, then said patient is responding to said treatment. This efficient treatment should therefore be preferentially maintained.

Conversely, if the BV patient is diagnosed to be "severe" before the administration of the treatment and remains "severe" upon administration of the treatment, then said patient is not responding to said treatment, and it is better to replace said treatment with another one or to combine it with another treatment.

Of note, if the BV patient is diagnosed to be "mild" before the administration of the treatment, then it is not worth administering any chemical treatment, as lifestyle interventions and pre, pro or postbiotics could be sufficient.

### Examples

### 1. Material and Methods

### 1.1. Cohort

### 1.1.1 BV & healthy cohort

All participants were part of the "CEBAM" randomized trial, NCT04989543, initiated in France in 2020. The aim was to study the Composition and Bacterial Diversity of the Vaginal Microbiota in Healthy Versus Pathological Conditions (Bacterial Vaginosis).

31 women with healthy vaginal microbiota (none of the Amsel criteria + Nugent score ≤3) and 30 women with BV (≥3 Amsel criteria + Nugent score ≥7) were included from three investigational sites.

### 1.1.3 Single patient longitudinal study

A single patient with BV was followed longitudinally (sampling over 4 months) to assess the effect of different treatments on her vaginal microbiome. This single case, was selected as an illustration of how a medical device can be used instead of an antibiotic treatment to improve the vaginal eco-system.

### 1.2. Sample collection and preparation

### 1.2.1. Vaginal sampling

The subjects from the CEBAM trial were sampled by a medical doctor during their visit at the gynecology clinic. Upon collection of a vaginal swab, immersed in 1mL of DNA preservation buffer (typically DNA/RNA Shield^{®}), the tubes containing the samples were shipped by regular post to the laboratory. The tube was stored at -20°C until DNA extraction.

The same collection kit was used for the control cohorts.

### 1.2.2. DNA extraction

The vaginal sample was unfrozen and then extracted using the ZymoBIOMICS^{™} DNA Miniprep Kit which is designed for purifying DNA from a wide array of sample inputs. The ZymoBIOMICS^{™} innovative lysis system eliminates bias associated with unequal lysis efficiencies of different organisms (e.g. Gramnegative/positive bacteria including endospores2, fungi, protozoans, algae, etc.), making it ideal for microbial community profiling. (ZymoResearch). The DNA integrity and concentration were evaluated by Nanoquant and Agilent.

### 1.3. Nanopore sequencing

The sequencing was performed at Luxia Scientific in an ISO13485 accredited laboratory on a MinION MK1NC sequencer. Briefly the rapid barcoding kit was used on a R9 flow cell (ONT). The base-calling was performed using the provider's software. The average number of reads per sample was about 120k.

### 1.4 Bioinformatics processing

The raw reads were processed using Luxia Scientific's in house pipeline. Briefly the pipeline is based on tools used on EPI2ME(c) (Oxford Nanopore Technologies, UK), and a compilation of internal scripts. It consists of quality controls, mapping to the human genome (hg38) and mapping of left-over reads to NCBI RefSeq. The human reads identification is based on classification success upon the RefSeq hg38 single-genome database, and is carried out after the quality control steps that include removing reads with a low quality score or very few bases. The percentage of human reads in a sample is calculated as the number of reads mapping to hg38 over the number of reads after quality and length filtering.

### 2. Results

### 2.1. Comparison between controls versus Bacterial Vaginosis patients (BV)

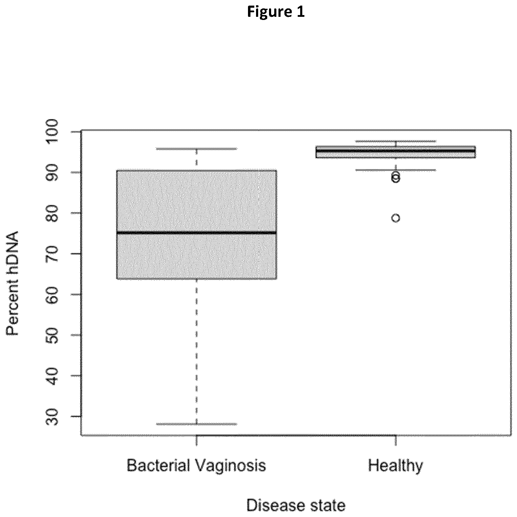

Using the T- test, 31 samples from healthy controls were compared to the 30 samples from BV patients. The p-value was highly significant for BV versus healthy controls (p-value = 7.96e-07), Figure 1. Summary statistics for the two cohorts are provided in Table 1.

**Table 1: Summary statistics for percent of reads mapping to the Human genome HG19.**

| **Cohort** | **Min.** | **1stQu.** | **Median** | **Mean** | **3rdQu.** | Max. |
|---|---|---|---|---|---|---|
| **BV** | 28.11% | 62.20% | 75.15% | 75.70% | 90.32% | 95.77% |
| **Healthy** | 78.77% | 93.6% | 95.28% | 94.03% | 96.32% | 97.6% |

None of the samples from healthy controls had less than 75% human DNA in their total vaginal DNA, compared to 50% of BV samples. A rate of human DNA below 75% is thus a marker of bacterial vaginosis.

### 2.2. Association to disease severity in dysbiotic patients

Since there is a highly significant difference between BV patients and healthy controls in terms of percent of human DNA in the vaginal sample, the relation to disease severity was studied, to assess whether the measure of host DNA relative abundance could be used as a biomarker of disease activity.

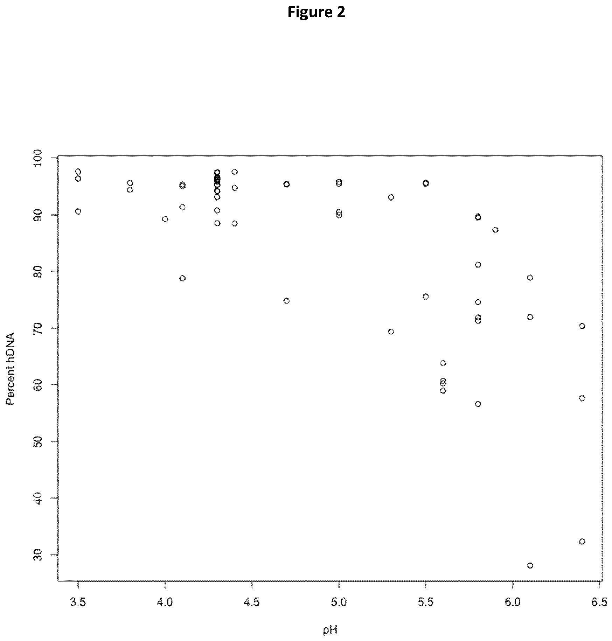

To that end, correlation was evaluated between pH (a marker of dysbiosis severity) and percent host DNA (Figure 2). A healthy state is considered when the pH is between 3.8 and 4.4. An acidic state (pH >4.4) is considered as a marker of vaginal dysbiosis. As can be seen, all samples with less than 75% hDNA come from acidic vagina. In particular samples with less than 65% hDNA come from an extremely acidic vagina (pH>5.5).

## Claims

1. An *in vitro* method for diagnosing Bacterial Vaginosis (BV) in a subject, said method comprising:
a) Obtaining a sample of vaginal microbiota from said subject,
b) Determining the relative abundance of host DNA in said sample,
and
c) Diagnosing that said subject suffers from Bacterial Vaginosis, if said relative abundance is lower than a reference value.

2. The method of claim 1, wherein said subject is a woman who is suspected of suffering from BV, or is in need of a confirmation of having BV.

3. The method of claim 1 or 2, wherein step b) comprises :
i) sequencing the DNA present in the sample using high throughput sequencing technologies and
ii) aligning the reads obtained by means of these sequencing technologies to the human genome.

4. The method of claim 1 or 2, wherein step b) comprises measuring host DNA abundance by qPCR with human specific primers.

5. The method of any of claims 1 to 4, wherein said reference value is of about 75%.

6. The method of any of claims 1 to 5, comprising the additional step of designing a treatment for the diagnosed subject.

7. The method of any of claims 1 to 5, comprising the additional step of detecting a severity stage of BV.

8. The method of any of claims 1 to 5, comprising the additional step of adapting a treatment to the particular state of BV which has been diagnosed.
